# FASCICULE DE BREVET EUROPEEN

(11) **EP 0 733 627 B1**
(45) Date de publication et mention de la délivrance du brevet: **28.06.2000**
(21) Numéro de dépôt: 96400586.2
(22) Date de dépôt: 21.03.1996
(51) Int. Cl.: C07D 333/22, A61K 31/38

(54) **Nouveaux composés du thiophene, leur procédé de préparation et les compositions pharmaceutiques qui les contiennent**
Thiophenverbindungen, Verfahren zu deren Herstellung und diese enthaltende pharmazeutische Zubereitungen
Thiophene compounds, a method for their preparation and pharmaceutical compositions containing them

(30) Priorité: 21.03.1995 FR 9503246
(43) Date de publication de la demande: 25.09.1996
(73) Titulaire: ADIR ET COMPAGNIE, 92415 Courbevoie Cédex (FR)
(72) Inventeur: Wierzbicki, Michel, 78620 L'Etang la Ville (FR); Boussard, Marie-Françoise, 78124 Mareil sur Mauldre (FR); Bonnet, Jacqueline, 75013 Paris (FR); Sabatini, Massimo, 92380 Garges (FR); Pastoureau, Philippe, 92310 Sevres (FR)
(74) Mandataire: Reverbori, Marcelle

(56) Documents cités:
- EP-A- 0 429 370
- JOURNAL OF MEDICINAL CHEMISTRY, vol. 27, no. 3, Mars 1984, WASHINGTON US, pages 390-397, XP002006148 M.M. GOODMAN ET AL.: "Synthesis and Evaluation of Radioiodinated Terminal p-Idophenyl-Substituted alpha- and beta-Methyl-Branched Fatty Acids"

## Description

La présente invention a pour objet de nouveaux composés du thiophène, leur procédé de préparation et les compositions pharmaceutiques qui en contiennent.

Elle concerne plus particulièrement les composés du thiophène de formule I: 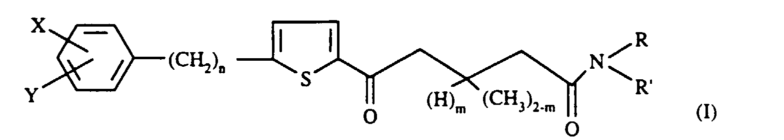 dans laquelle :
- X et Y, identiques ou différents, représentent chacun un atome d'hydrogène ou d'halogène, un radical alkyle ou alkoxy ayant chacun de 1 à 5 atomes de carbone en chaîne droite ou ramifiée, ou un radical dialkylamino dans lequel chaque groupe alkyle renferme de 1 à 5 atomes de carbone en chaîne droite ou ramifiée ;
- n représente un nombre entier de 2 à 5 inclus ;
- m représente zéro, un ou deux ;
- R représente un atome d'hydrogène ou un radical alkyle ayant de 1 à 5 atomes de carbone en chaîne droite ou ramifiée ; et
- R' représente un radical alkyle ayant de 1 à 5 atomes de carbone en chaîne droite ou ramifiée ; ou
- R et R' forment ensemble avec l'atome d'azote auquel ils sont liés un radical hétérocyclique penta ou hexagonal renfermant éventuellement un atome d'oxygène ou un deuxième atome d'azote, lequel radical hétérocyclique est soit non substitué soit substitué par un radical alkyle ayant de 1 à 5 atomes de carbone en chaîne droite ou ramifiée, ou par un radical arylalkyle dans lequel le groupe alkyle renferme de 1 à 5 atomes de carbone en chaîne droite ou ramifiée et le groupe aryle (qui est préférentiellement phényle) est soit non substitué, soit substitué par un ou plusieurs substituants identiques ou différents choisis parmi les atomes d'halogène et les radicaux alkyle et alkoxy ayant chacun de I à 5 atomes de carbone en chaîne droite ou ramifiée.
Lorsque m prend la valeur 1, les composés de formule I renferment un carbone asymétrique et de ce fait existent sous forme d'énantiomères qui font, à ce titre, partie de la présente invention.
Les composés de formule I qui renferment une fonction amine libre (c'est à dire autre que celle liée au groupe carbonyle) donnent des sels avec les acides physiologiquement tolérables -sels qui sont également, à ce titre, inclus dans la présente invention.

L'état antérieur de la technique le plus proche de la présente invention est illustré par le brevet EP 0 429 370 qui concerne, entre autres, les 5-phénylalkyl 2-aminoalkyl thiophènes de formule : 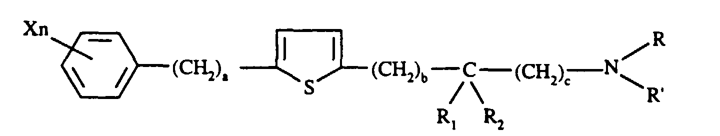 ayant une activité anti-résorbante osseuse.

Les modifications de structure qui, à partir de ces dits composés antérieurement connus -et en particulier l'introduction, dans la chaîne amino alkyle, de deux groupements carbonyles- a conduit aux composés de la présente invention qui, outre le fait qu'ils soient nouveaux par rapport aux composés de l'état antérieur de la technique, présentent par rapport à ces derniers des avantages pharmacologiques importants, notamment une meilleure hydrosolubilité et un potentiel anti-résorbant accru, ce qui permet de les utiliser pour un même effet thérapeutique à moindre risque (le caractère hydrophile des produits diminuant les effets secondaires notamment dans la sphère gastro-entérologique) et à moindre coût (grâce à l'utilisation de posologies plus faibles).

La présente invention a également pour objet le procédé de préparation des composés de formule I caractérisé en ce que l'on effectue le couplage :
- d'un acide de formule II : 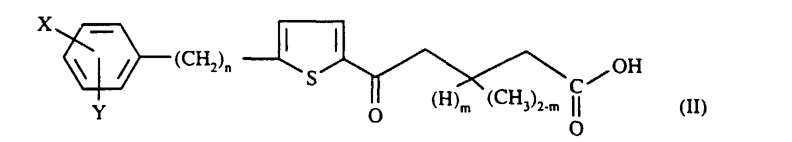 dans laquelle X, Y, n et m ont les significations précédemment définies,
- et d'une amine de formule III : 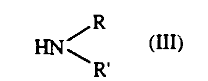 dans laquelle R et R' ont les significations précédemment définies,
au moyen d'un agent couplant dans un solvant approprié.

Il est particulièrement judicieux d'utiliser le carbonyl di-imidazole comme agent couplant et d'effectuer la réaction dans le dichlorométhane.
Les amines de formule III utilisées comme matières premières sont des produits connus.
Les acides de formule II utilisés comme matières premières sont soit connus, soit préparés, à partir de substances connues, selon des procédés utilisés pour la synthèse d'acides analogues, comme indiqué dans les exemples ci-après.

Les dérivés de la présente invention possèdent des propriétés pharmacologiques et thérapeutiques intéressantes notamment sur le métabolisme osseux.

A l'état normal, le remodelage osseux permet d'assurer l'intégrité anatomique et structurale du squelette. Il s'opère par cycles et dépend de deux grands types de population cellulaire, les ostéoclastes et les ostéoblastes. Les ostéoclastes, après activation, assurent la résorption de l'os ancien par acidification puis digestion enzymatique, laissant place, dans une phase successive, aux ostéoblastes pour former le nouveau tissu ostéoïde qui sera ensuite calcifié. Ces deux phases métaboliques -résorption et formation- sont à l'état physiologique étroitement couplées l'une à l'autre. En situation pathologique, un déséquilibre de ces deux phases peut se produire, avec en particulier une hyperactivité de résorption entrainant une perte osseuse excessive insuffisamment compensée par la phase de neoformation. C'est en particulier le cas de l'ostéoporose post-ménopausique, de la maladie de Paget et de l'hypercalcémie d'origine maligne.

Les composés de la présente invention possèdent des propriétés pharmacologiques et thérapeutiques particulièrement intéressantes en raison de leur activité inhibitrice de l'hyperrésorption osseuse, laquelle activité a été mise en évidence à la fois in vitro et in vivo comme le prouve l'étude pharmacologique ci-après décrite dans l'exemple 27.

Ces propriétés pharmacologiques permettent l'utilisation des composés de la présente invention dans les pathologies caractérisées par une perte osseuse due à une hyperactivité de résorption comme en particulier l'ostéoporose post-ménopausique, la maladie de Paget et l'hypercalcémie maligne.

La présente invention a également pour objet les compositions pharmaceutiques contenant comme principe actif un composé de formule I ou un de ses sels physiologiquement tolérable, mélangé ou associé à un excipient pharmaceutique approprié comme, par exemple, le glucose, le lactose, l'amidon, le talc, l'éthyl cellulose, le stéarate de magnésium ou le beurre de cacao. Les compositions pharmaceutiques ainsi obtenues se présentent généralement sous forme dosée et peuvent revêtir, par exemple, la forme de comprimés, dragées, gélules, suppositoires, solutions injectables ou buvables et être selon les cas administrées par voie orale, rectale, ou parentérale.
La posologie varie selon l'âge et le poids du patient, la voie d'administration et les traitements associés.

Les exemples suivants illustrent la présente invention.

### Exemple 1

5-[4-(4-méthylphényl)butyl] 2-{3,3-diméthyl 4-[N'-(2,3,4-triméthoxybenzyl)N-pipérazinyl carbonyl]butyryl}thiophène. 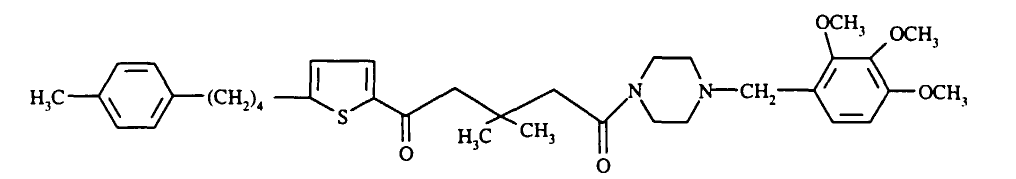 43,3 g (0,116 mole) d'acide 5-[4-(4-méthylphényl)butyl] 3,3-diméthyl 4-{5-[4-(4-méthylphényl)butyl]thién-2-yl carbonyl}butyrique (préparé selon la méthode décrite dans le brevet EP 0429370) sont dissouts dans 200 ml de dichlorométhane. On ajoute alors lentement à cette solution, que l'on refroidit à 10 °C, 26 g (0,16 mole) de carbonyl diimidazole puis on laisse revenir le milieu à température ambiante et maintient l'agitation jusqu'à réaction complète et fin du dégagement gazeux.
On ajoute ensuite lentement au milieu réactionnel 58,81 g (0,22 mole) de N-(2,3,4-triméthoxybenzyl)pipérazine en solution dans 200 ml de dichlorométhane. L'ensemble est maintenu à température ambiante sous agitation pendant 24 heures, puis additionné de 500 ml d'eau. Après décantation, la phase organique est lavée successivement par 200 ml d'eau puis par 2 fois 100 ml d'une solution 0,1 N d'HCl.
La phase organique est ensuite amenée à sec par distillation. Le résidu est purifié par chromatographie sur silice en éluant au dichlorométhane. Les fractions sont rassemblées et après élimination du solvant, on obtient le produit attendu sous forme d'huile.
La base ainsi obtenue est dissoute dans 100 ml d'éther anhydre, et additionnée de 30 ml d'une solution 4N d'HCl dans l'éther. Le précipité formé est filtré puis repris dans le minimum d'acétate d'éthyle. La solution est filtrée sur millipore et le filtrat est amené à sec. Le résidu est recristallisé dans l'isopropanol anhydre. On obtient ainsi 50,5 g du chlorhydrate de 5-[4-(4-méthyl phényl)butyl] 2-{3,3-diméthyl 4-[N'-(2,3,4-triméthoxybenzyl) N-pipérazinyl carbonyl] butyryl} thiophène, PF : 96 °C.

### Exemples 2 à 26

En opérant comme décrit dans l'exemple 1 ont été préparés les dérivés objet des exemples suivants :
2) 5-[3-(4-méthylphényl)propyl] 2-{3,3-diméthyl 4-[N'-(2,3,4-triméthoxybenzyl) N-pipérazinyl carbonyl]butyryl}thiophène, (huile), dont le chlorhydrate fond à 70 °C.
3) 5-[3-(4-méthylphényl)propyl] 2-{4-[N'-(2,3,4-triméthoxybenzyl) N-pipérazinyl carbonyl] butyryl}thiophène (huile), dont le chlorhydrate fond à 138 °C.
4) 5-[3-(4-isopropylphényl)propyl] 2-{3,3-diméthyl 4-[N'-(2,3,4-triméthoxybenzyl) N-pipérazinyl carbonyl]butyryl}thiophène, (huile), dont le chlorhydrate fond à 94 °C.
5) 5-[2-(4-méthylphényl)éthyl] 2-{3,3-diméthyl-4-[N'-(2,3,4-triméthoxybenzyl) N-pipérazinyl carbonyl]butyryl} thiophène, (huile), dont le chlorhydrate fond à 107 °C.
6) 5-[3-(2,5-diméthylphényl)propyl] 2-{3,3-diméthyl 4-[N'-(2,3,4-triméthoxybenzyl) N-pipérazinyl carbonyl]butyryl}thiophène, (huile), dont le chlorhydrate fond à 89 °C.
7) 5-[5-(4-méthylphényl)pentyl] 2-{3,3-diméthyl 4-[N'-(2,3,4-triméthoxybenzyl) N-pipérazinyl carbonyl]butyryl}thiophène, (huile), dont le chlorhydrate fond à 99-100 °C.
8) 5-[3-(2,4-diméthylphényl)propyl] 2-{3,3-diméthyl 4-[N'-(2,3,4-triméthoxybenzyl) N-pipérazinyl carbonyl]butyryl}thiophène, (huile), dont le chlorhydrate fond à 118 °C.
9) 5-[4-(4-méthylphényl)butyl] 2-{3-méthyl 4-[N'-(2,3,4-triméthoxybenzyl) N-pipérazinyl carbonyl]butyryl}thiophène, (huile), dont le chlorhydrate fond à 135 °C.
10) 5-[4-(4-méthylphényl)butyl] 2-{4-[N'-(2,3,4-triméthoxybenzyl) N-pipérazinyl carbonyl] butyryl}thiophène, (huile), dont le chlorhydrate fond à 138-140 °C.
11) 5-[3-(4-méthylphényl)propyl] 2-{3-méthyl 4-[N'-(2,3,4-triméthoxybenzyl) N-pipérazinyl carbonyl]butyryl}thiophène, (huile), dont le chlorhydrate fond à 118 °C.
12) 5-[3-(4-isopropylphényl)propyl] 2-(3,3-diméthyl 4-diéthylamino carbonyl)butyryl thiophène, (huile), ν CO : 1643 cm⁻¹.
13) 5-[3-(2,5-diméthylphényl)propyl] 2-(3,3-diméthyl 4-morpholino carbonyl butyryl) thiophène, (huile), ν CO : 1644 cm⁻¹.
14) 5-[5-(4-méthyl phényl)pentyl] 2-(3,3-diméthyl 4-pipéridino carbonyl butyryl)thiophène, (huile), ν CO : 1644 cm⁻¹.
15) 5-[3-(4-isopropylphényl)propyl] 2-(4-morpholino carbonyl butyryl)thiophène, (huile), ν CO : 1652 cm⁻¹.
16) 5-[2-(4-méthylphényl)éthyl] 2-{3,3-diméthyl 4-[N'-(2-cyclopentyloxy 3,4-diméthoxy benzyl) N-pipérazinyl carbonyl]butyryl}thiophène, (huile), ν CO du chlorhydrate : 1645 cm⁻¹, préparé en utilisant, comme amine, la N-(2-cyclopentyloxy 3,4-diméthoxy benzyl)pipérazine, elle même préparée par alcoylation de la N-benzyl N'-(2-hydroxy 3,4-diméthoxy benzyl) pipérazine avec du bromocyclopentane, suivie d'une débenzylation catalytique.
17) 5-[3-(4-méthylphényl)propyl] 2-(3,3-diméthyl 4-diéthyl amino carbonyl butyryl) thiophène, (huile), ν CO : 1642 cm⁻¹.
18) 5-[3-(4-isopropylphényl)propyl] 2-[3,3-diméthyl 4-(N-méthyl N-cyclohexyl amino carbonyl)butyryl]thiophène, (huile), ν CO : 1643 cm⁻¹.
19) 5-[3-(2,5-diméthylphényl)propyl] 2-[3,3-diméthyl 4-(N'-méthyl N-pipérazinyl carbonyl) butyryl]thiophène, (huile), dont le chlorhydrate fond à 120°C.
20) 5-[5-(4-méthylphényl)pentyl] 2-{3,3-diméthyl 4-[N'-(2,4-diméthoxy 3-propyloxy benzyl) N-pipérazinyl carbonyl]butyryl}thiophène, (huile), ν CO du chlorhydrate : 1646 cm⁻¹, préparé en utilisant, comme amine, la N-(2,4-diméthoxy 3-n-propyloxy benzyl)pipérazine, elle-même préparée par alcoylation de la N-benzyl N'-(2,4-diméthoxy 3-hydroxy benzyl)pipérazine, suivie d'une hydrogénation catalytique.
21)5-[3-(2,5-diméthyl phényl)propyl] 2-{3-méthyl 4-[N'-(3,4-diméthoxy 2-pentyloxy benzyl) N-pipérazinyl carbonyl]butyryl}thiophène, (huile), ν CO du chlorhydrate : 1651 cm⁻¹, préparé en utilisant, comme amine, la N-(2-n-pentyloxy 3,4-diméthoxy benzyl)pipérazine, elle-même préparée par alcoylation de la N-benzyl N'-(2-hydroxy 3,4-diméthoxy benzyl)pipérazine avec du 1-bromopentane, suivie d'une débenzylation catalytique.
22) 5-[3-(4-isopropylphényl)propyl] 2-[4-(N'-méthyl N-pipérazinyl carbonyl) butyryl] thiophène, (huile), dont le chlorhydrate fond à 162°C.
23) 5-[3-(2,5-diméthyl phényl) propyl] 2-[3-méthyl 4-(N-méthyl N-cyclohexyl aminocarbonyl) butyryl] thiophène, (huile), ν CO : 1642 cm⁻¹.
24) 5-[4-(4-méthyl phényl) butyl] 2-[3-méthyl 4-(diéthylaminocarbonyl) butyryl] thiophène, (huile), ν CO : 1644 cm⁻¹.
25) (R)-5-[4-(4-méthyl phényl) butyl] 2-{3-méthyl 4-[N'-(2,3,4-triméthoxybenzyl) N-pipérazinyl carbonyl] butyry} thiophène, (huile), dont le chlorhydrate fond à 127°C.
26) (S)-5-[4-(4-méthyl phényl) butyl] 2-{3-méthyl 4-[N'-(2,3,4-triméthoxybenzyl) N-pipérazinyl carbonyl] butyryl} thiophène, (huile), dont le chlorhydrate fond à 127°C.

Les acides de formule II utilisés comme matières premières pour synthétiser les produits ci-dessus exemplifiés ont été préparés comme suit.
1- Les acides de formule II dans laquelle m prend soit la valeur zéro, soit la valeur 2 ont été préparés selon la méthode décrite dans le brevet EP 0 429 370 pour la synthèse des composés de formule (C) de ce dit brevet.
2- Les acides de formule dans laquelle m prend la valeur 1, qui sont des acides asymétriques, ont été préparés comme décrit ci-après pour la préparation de l'acide (R) 5-{5-[4-(4-méthylphényl)butyl]thién-2-yl } 5-oxo 3-méthyl pentanoïque

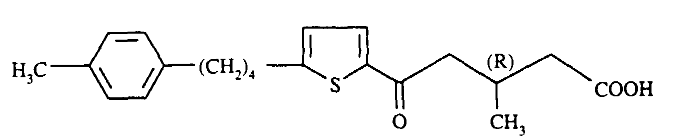

3,45 g (0,015 mole) de 2-[4-(4-méthylphényl)butyl]thiophène, sont mis en solution dans 20 ml de tétrahydrofurane. La solution obtenue est refroidie à - 70 °C puis additionnée goutte à goutte de 12 ml d'une solution 1,6 M de Bu Li dans le tétrahydrofurane. On laisse le milieu revenir à 0 °C, et ajoute lentement ce mélange à une solution de 4,18 g (0,0194 mole) de bromure de manganèse dans 30 ml de tétrahydrofurane à 0 °C. On laisse revenir le milieu à 20 °C et le maintient ainsi pendant 30 minutes, puis le refroidit à - 10 °C et l'additionne alors à du chlorure d'acide de formule : 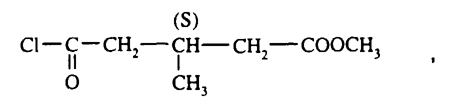 en solution dans 10 ml d'éther éthylique (lequel chlorure d'acide a été préparé par addition de 2 ml de chlorure d'oxalyle à une solution de 2,4 g (0,015 mole) de (R)-(+)-3-méthyl mono glutarate de méthyle dans 2 ml de chloroforme, suivie d'une agitation à température ambiante pendant 2 heures puis élimination des fractions volatiles).
Le milieu réactionnel est maintenu 15 minutes à - 10 °C puis 12 heures à 20 °C. On l'hydrolyse avec 40 ml d'eau et glace et 20 ml d'HCl, N. Après décantation, lavage à l'eau puis avec une solution à 10 % de NaHCO₃, la phase éthérée est séchée sur sulfate de magnésium. Après distillation de l'éther sous vide et chromatographie du résidu sur silice en éluant avec cyclohexane/dichlorométhane (80/20), on obtient 4,2 g de (R) 5-{5-[4-(4-méthylphényl)butyl] thién-2-yl} 5-oxo 3-méthyl pentanoate de méthyle à l'état pur.
Les 4,2 g d'ester ainsi obtenu sont mis en solution dans un mélange de 15 ml d'une solution N de soude et 15 ml d'éthanol. La solution est portée puis maintenue à reflux pendant 30 minutes, puis l'éthanol est distillé sous pression réduite. La phase aqueuse est refroidie à 0 °C puis acidifiée par 15 ml d'une solution N d'HCl. Le précipité formé est filtré et séché. On obtient ainsi 3,6 g d'acide (R) 5-{5-[4-(4-méthylphényl)butyl]thién-2-yl} 5-oxo 3-méthyl pentanoïque utilisé tel quel pour la synthèse ultérieure, (échantillon analytique, PF : 94 °C).

De la même manière a été préparé l'acide (S) correspondant à partir du (S)-(-)-3-méthyl mono glutarate de méthyle.

### Exemple 27

### Etude pharmacologique

L'activité inhibitrice de l'hyperrésorption osseuse des composés de la présente invention a été mise en évidence comme suit :

### 1. In vitro

Cette activité a été montrée sur un test d'hyperrésorption pratiqué sur des cultures de tissu osseux de souris (calvaria) selon une technique inspirée de la méthode de J.J. Reynolds et coll. *Calc. Tiss. Res.* 4, *339-349 (1970).* Sur l'hyperrésorption induite par différents agents -acide rétinoïque, PTH- les composés testés ont éxercé une puissante inhibition.
A titre d'exemple, les résultats obtenus sur l'hyperrésorption à l'acide rétinoïque avec un ensemble de composés représentatifs de l'invention sont rassemblés dans le tableau ci-après :

| **Composés** | **Hyperrésorption à l'acide rétinoïque** **% d'inhibition de l'hyperrésorption à la dose de 1 µM** |
|---|---|
| Exemple 1 | 74,2 ± 2,3 % |
| Exemple 3 | 28,3 ± 7,3 % |
| Exemple 4 | 14,3 ± 10,4 % |
| Exemple 7 | 67,6 ± 5,1 % |
| Exemple 9 | 81,5 ± 2,2 % |
| Exemple 10 | 75,1 ± 3,6 % |

Les IC₅₀ des composés des exemples 1, 7 et 9 sont respectivement de 0,50, 0,37 et 0,27 µM.
Les activités des composés des exemples 25 et 26 sont du même ordre de grandeur que celle du composé de l'exemple 9.

### 2. In vivo

Cette activité a été mise en évidence chez la souris placée dans une situation d'hyperrésorption osseuse induite par un puissant arotinoïde -le Ro 13-6298-, selon une méthodologie inspirée des travaux de U. Treschel et coll. *J. Clin. Invest. 80*, *1679-1686 (1987).* Par exemple, administrés par voie orale quotidiennement pendant 5 jours, aux doses de 0,5 et 1 mg/kg, les composés de la présente invention objet des exemples 1, 7 et 9- s'opposent à l'hypercalcémie faisant suite à l'hyperrésorption arotinoïque.

En conséquence, les composés de la présente invention pourront être utilisés dans le traitement des pathologies caractérisées par une perte osseuse due à une hyperactivité de résorption comme en particulier l'ostéoporose post-ménopausique, la maladie de Paget et l'hypercalcémie maligne.

## Revendications

1. Les composés du thiophène de formule I : 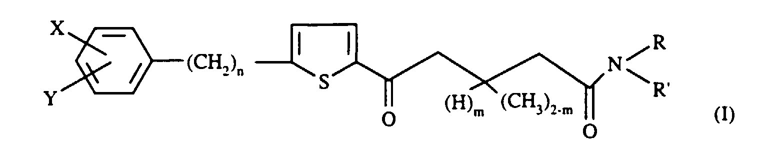 dans laquelle :
- X et Y, identiques ou différents, représentent chacun un atome d'hydrogène ou d'halogène, un radical alkyle ou alkoxy ayant chacun de 1 à 5 atomes de carbone en chaîne droite ou ramifiée, ou un radical dialkylamino dans lequel chaque groupe alkyle renferme de 1 à 5 atomes de carbone en chaîne droite ou ramifiée ;
- n représente un nombre entier de 2 à 5 inclus ;
- m représente zéro, un ou deux ;
- R représente un atome d'hydrogène ou un radical alkyle ayant de 1 à 5 atomes de carbone en chaîne droite ou ramifiée ; et
- R' représente un radical alkyle ayant de 1 à 5 atomes de carbone en chaîne droite ou ramifiée ; ou
- R et R' forment ensemble avec l'atome d'azote auquel ils sont liés un radical hétérocyclique penta ou hexagonal renfermant éventuellement un atome d'oxygène ou un deuxième atome d'azote, lequel radical hétérocyclique est soit non substitué soit substitué par un radical alkyle ayant de 1 à 5 atomes de carbone en chaîne droite ou ramifiée, ou par un radical arylalkyle dans lequel le groupe alkyle renferme de 1 à 5 atomes de carbone en chaîne droite ou ramifiée et le groupe aryle est soit non substitué, soit substitué par un ou plusieurs substituants identiques ou différents choisis parmi les atomes d'halogène et les radicaux alkyle et alkoxy ayant chacun de 1 à 5 atomes de carbone en chaîne droite ou ramifiée,
ainsi que, quand ils existent :
- les énantiomères correspondants, et
- leurs sels d'addition avec des acides physiologiquement tolérables.

2. Un composé de la revendication 1 choisi parmi le 5-[4-(4-méthylphényl)butyl] 2-{3,3diméthyl 4-[N'-(2,3,4-triméthoxybenzyl N-pipérazinyl carbonyl]butyryl}thiophène, et son chlorhydrate.

3. Un composé de la revendication 1 choisi parmi le 5-[5-(4-méthylphényl)pentyl] 2-{3,3-diméthyl 4-[N'-(2,3,4-triméthoxybenzyl) N-pipérazinyl carbonyl]butyryl}thiophène, et son chlorhydrate.

4. Un composé de la revendication 1 choisi parmi le 5-[4-(4-méthylphényl)butyl] 2-{3-méthyl 4-[N'-(2,3,4-triméthoxybenzyl) N-pipérazinyl carbonyl]butyryl}thiophène, et son chlorhy drate.

5. Un composé de la revendication 1 choisi parmi le 5-[4-(4-méthylphényl)butyl] 2-{4-[N'-(2,3,4-triméthoxybenzyl) N-pipérazinyl carbonyl]butyryl}thiophène, et son chlorhydrate.

6. Un composé de la revendication 1 choisi parmi le (R)-5-[4-(4-méthylphényl)butyl] 2-{3-méthyl 4-[N'-(2,3,4-triméthoxybenzyl) N-pipérazinyl carbonyl]butyryl}thiophène, et son chlorhydrate.

7. Un composé de la revendication 1 choisi parmi le (S)-5-[4-(4-méthylphényl)butyl] 2-{3-méthyl 4-[N'-(2,3,4-triméthoxybenzyl) N-pipérazinyl carbonyl]butyryl}thiophène, et son chlorhydrate.

8. Le procédé de préparation des composés de la revendication 1 caractérisé en ce que l'on effectue le couplage :
- d'un acide de formule II : 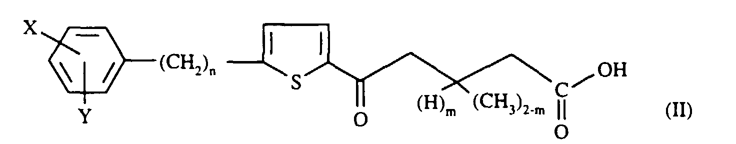 dans laquelle X, Y, n et m ont les significations définies dans la revendication 1,
- et d'une amine de formule III : 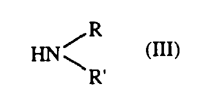 dans laquelle R et R' ont les significations définies dans la revendication 1,
au moyen d'un agent couplant dans un solvant approprié ;
et si on le désire, on transforme les composés ainsi obtenus qui renferment une fonction amine libre en leurs sels d'addition avec des acides physiologiquement tolérables.

9. A titre de médicament, un composé selon l'une quelconque des revendications 1 à 7,

10. Les compositions pharmaceutiques, utilisables dans le traitement des pathologies caractérisées par une perte osseuse due à une hyperactivité de résorption, contenant comme principe actif au moins un des composés selon l'une quelconque des revendications 1 à 7, seul ou en combinaison avec un ou plusieurs excipients pharmaceutiquement appropriés

## Claims

1. Thiophene compounds of formula I: 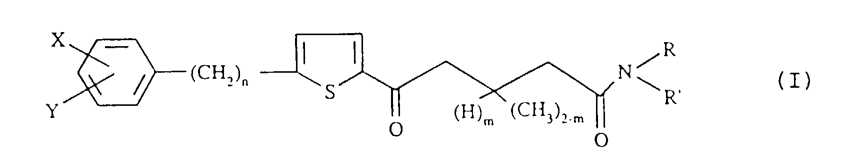 in which:
- each of X and Y, which are the same or different, represents a hydrogen or halogen atom, an alkyl or alkoxy radical each having from 1 to 5 carbon atoms in a straight or branched chain, or a dialkylamino radical in which each alkyl group contains from 1 to 5 carbon atoms in a straight or branched chain;
- n represents an integer from 2 to 5 inclusive;
- m represents zero, one or two;
- R represents a hydrogen atom or an alkyl radical having from 1 to 5 carbon atoms in a straight or branched chain; and
- R' represents an alkyl radical having from 1 to 5 carbon atoms in a straight or branched chain; or
- R and R' form together with the nitrogen atom to which they are bonded a pentagonal or hexagonal heterocyclic radical optionally containing an oxygen atom or a second nitrogen atom, which heterocyclic radical is unsubstituted or substituted by an alkyl radical having from 1 to 5 carbon atoms in a straight or branched chain or by an arylalkyl radical in which the alkyl group contains from 1 to 5 carbon atoms in a straight or branched chain and the aryl group is unsubstituted or substituted by one or more identical or different substituents selected from halogen atoms and alkyl and alkoxy radicals each having from 1 to 5 carbon atoms in a straight or branched chain, and, where they exist,
- the corresponding enantiomers, and
- their addition salts with physiologically tolerable acids.

2. A compound of claim 1 selected from 5-[4-(4-methylphenyl)butyl]-2-{3,3-dimethyl-4-[N'-(2,3,4-trimethoxybenzyl)-N-piperazinylcarbonyl]butyryl}thiophene and its hydrochloride.

3. A compound of claim 1 selected from 5-[5-(4-methylphenyl)pentyl]-2-{3,3-dimethyl-4-[N'-(2,3,4-trimethoxybenzyl)-N-piperazinylcarbonyl]butyryl}thiophene and its hydrochloride.

4. A compound of claim 1 selected from 5-[4-(4-methylphenyl)butyl]-2-{3-methyl-4-[N'-(2,3,4-trimethoxybenzyl)-N-piperazinylcarbonyl]butyryl}thiophene and its hydrochloride.

5. A compound of claim 1 selected from 5-[4-(4-methylphenyl)butyl]-2-{4-[N'-(2,3,4-trimethoxybenzyl)-N-piperazinylcarbonyl]butyryl}thiophene and its hydrochloride.

6. A compound of claim 1 selected from (R)-5-[4-(4-methylphenyl)butyl]-2-{3-methyl-4-[N'-(2,3,4-trimethoxybenzyl)-N-piperazinylcarbonyl]butyryl}thiophene and its hydrochloride.

7. A compound of claim 1 selected from (S)-5-[4-(4-methylphenyl)butyl]-2-{3-methyl-4-[N'-(2,3,4-trimethoxybenzyl)-N-piperazinylcarbonyl]butyryl}thiophene and its hydrochloride.

8. Process for the preparation of compounds of claim 1, characterised in that:
- an acid of formula II: 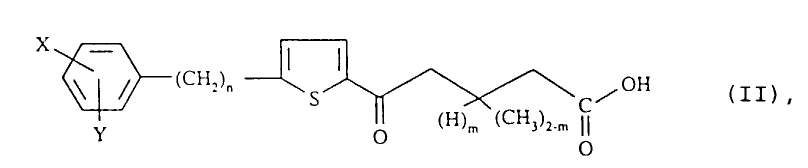 in which X, Y, n and m are as defined in claim 1,
- and an amine of formula III: 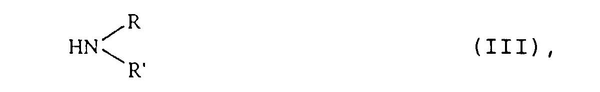 in which R and R' are as defined in claim 1,
are coupled by means of a coupling agent in a suitable solvent;
and, if desired, the compounds so obtained that contain a free amine function are converted into their addition salts with physiologically tolerable acids.

9. A compound according to any one of claims 1 to 7 as a medicament.

10. Pharmaceutical compositions, for use in the treatment of pathologies characterised by bone loss due to resorptive hyperactivity, comprising as active ingredient at least one of the compounds according to any one of claims 1 to 7, alone or in combination with one or more suitable pharmaceutical excipients.

## Patentansprüche

1. Thiophenverbindungen der Formel I: 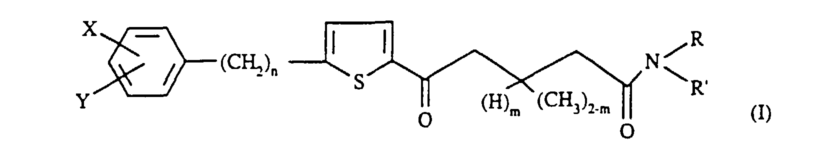 in der:
- X und Y, die gleichartig oder verschieden sind, jeweils ein Wasserstoffatom oder ein Halogenatom, eine Alkyl- oder Alkoxygruppe mit jeweils 1 bis 5 Kohlenstoffatomen in gerader oder verzweigter Kette oder eine Dialkylaminogruppe, in der jede Alkylgruppe 1 bis 5 Kohlenstoffatome in gerader oder verzweigter Kette umfaßt;
- n eine ganze Zahl mit einem Wert von 2 bis 5 einschließlich;
- m Null, Eins oder Zwei;
- R ein Wasserstoffatom oder eine Alkylgruppe mit 1 bis 5 Kohlenstoffatomen in gerader oder verzweigter Kette; und
- R' eine Alkylgruppe mit 1 bis 5 Kohlenstoffatomen in gerader oder verzweigter Kette; oder
- R und R' gemeinsam mit dem Stickstoffatom, an das sie gebunden sind, einen penta- oder hexagonalen heterocyclischen Rest, der gegebenenfalls ein Sauerstoffatom oder ein zweites Stickstoffatom enthält, welcher heterocyclische Rest entweder unsubstituiert ist oder durch eine Alkylgruppe mit 1 bis 5 Kohlenstoffatomen in gerader oder verzweigter Ketter oder durch eine Arylalkylgruppe substituiert ist, worin die Alkylgruppe 1 bis 5 Kohlenstoffatome in gerader oder verzweigter Kette aufweist und die Arylgruppe entweder unsubstituiert ist oder durch ein oder mehrere gleichartige oder verschiedene Substituenten ausgewählt aus Halogenatomen und Alkyl- und Alkoxygruppen mit jeweils 1 bis 5 Kohlenstoffatomen in gerader oder verzweigter Kette substituiert ist, bedeuten,
sowie, falls sie existieren:
- die entsprechenden Enantiomeren und
- ihre Additionssalze mit physiologisch verträglichen Säuren.

2. Verbindung nach Anspruch 1 ausgewählt aus 5-[4-(4-Methylphenyl)-butyl]-2-{3,3-dimethyl-4-[N'-(2,3,4-trimethoxybenzyl)-N-piperazinyl-carbonyl]-butyryl}-thiophen und dessen Hydrochlorid.

3. Verbindung nach Anspruch 1 ausgewählt aus 5-[5-(4-Methylphenyl)-pentyl]-2-{3,3-dimethyl-4-[N'-(2,3,4-trimethoxybenzyl)-N-piperazinyl-carbonyl]-butyryl}-thiophen und dessen Hydrochlorid.

4. Verbindung nach Anspruch 1 ausgewählt aus 5-[4-(4-Methylphenyl)-butyl]-2-{3-methyl-4-[N'-(2,3,4-trimethoxybenzyl)-N-piperazinyl-carbonyl]-butyryl}-thiophen und dessen Hydrochlorid.

5. Verbindung nach Anspruch 1 ausgewählt aus 5-[4-(4-Methylphenyl)-butyl]-2-(4-[N'-(2,3,4-trimethoxybenzyl)-N-piperazinyl-carbonyl]-butyryl}-thiophen und dessen Hydrochlorid.

6. Verbindung nach Anspruch 1 ausgewählt aus (R)-5-[4-(4-Methylphenyl)-butyl]-2-{3-methyl-4-[N'-(2,3,4-trlmethoxybenzyl)-N-piperazinyl-carbonyl]-butyryl}-thiophen und dessen Hydrochlorid.

7. Verbindung nach Anspruch 1 ausgewählt aus (S)-5-[4-(4-Methylphenyl)-butyl]-2-{3-methyl-4-[N'-(2,3,4-trimethoxybenzyl)-N-piperazinyl-carbonyl]-butyryl}-thiophen und dessen Hydrochlorid.

8. Verfahren zur Herstellung der Verbindungen nach Anspruch 1, dadurch gekennzeichnet, daß man die Kupplung:
- einer Säure der Formel II: 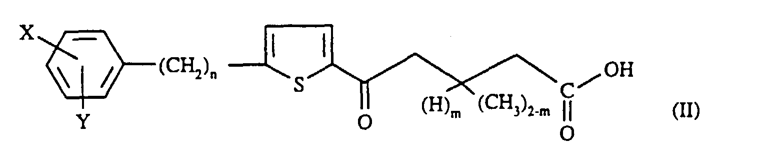 in der X, Y, n und m die in Anspruch 1 angegebenen Bedeutungen besitzen, mit einem Amin der Formel III: 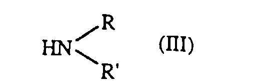 in der R und R' die in Anspruch 1 angegebenen Bedeutungen besitzen, mit Hilfe eines Kupplungsmittels in einem geeigneten Lösungsmittel bewirkt; und gewünschtenfalls die in dieser Weise erhaltenen Verbindungen, welche eine freie Aminfunktion aufweisen, in ihre Additionssalze mit physiologisch verträglichen Säuren überführt.

9. Verbindung nach einem der Ansprüche 1 bis 7 als Arzneimittel.

10. Pharmazeutische Zubereitungen zur Behandlung von pathologischenZuständen gekennzeichnet durch einen Knochenverlust als Folge einer Resorptions-Hyperaktivität, enthaltend als Wirkstoff mindestens eine der Verbindungen nach einem der Ansprüche 1 bis 7 allein oder in Kombination mit einem oder mehreren pharmazeutisch geeigneten Trägermaterialien.
